# EUROPEAN PATENT APPLICATION

(11) **EP 2 087 919 A1**
(43) Date of publication of application: **12.08.2009**
(21) Application number: 09159905.0
(22) Date of filing: 16.06.2000
(51) Int. Cl.: A61M 16/06

(54) **Mask and headgear with connector**

(30) Priority: 18.06.1999 AU PQ104099; 18.06.1999 AU PQ191699; 16.12.1999 US 115618; 15.02.2000 US 504220
(62) Divisional of application: 05026584.2
(71) Applicant: ResMed Ltd., North Ryde, New South Wales 2113 (AU)
(72) Inventor: Gunaratnam, Michael Kassipillai, Marsfield, New South Wales 2122 (AU); Kwok, Philip Rodney, Chatswood, New South Wales 2113 (AU)
(74) Representative: Davies, Gregory Mark

(57) **Abstract**

A respiratory mask and headgear combination comprises a respiratory mask and adjustable headgear for securing the mask on a patient, the mask having a first connector portion (470) to receive a second connector portion when at least partially inserted into it to form a snap-fit locked connection, a press release mechanism being provided to allow single-handed disengagement by the patient in use.

## Description

### Field of the Invention

The invention relates to a connector for use with a mask and headgear suitable for the delivery of breathable gases to a patient for the treatment of sleep disordered breathing (SDB).

### Background of the Invention

Respiratory masks used in the treatment of SDB may comprise either a nasal mask, designed to fit over a patient's nose, or a full face mask designed to fit over the nose and mouth of the patient. Air or other breathable gas is supplied by a blower and passed along a flexible conduit to the mask.

The mask generally comprises a relatively rigid shell, termed a frame, which defines a rearwardly opening cavity covering the patient's nose and/or mouth and a soft portion, termed a cushion, which spaces the frame away from the face for comfortable contact.

The masks are typically held in place using headgear, the mask and headgear being joined using some form of connector.

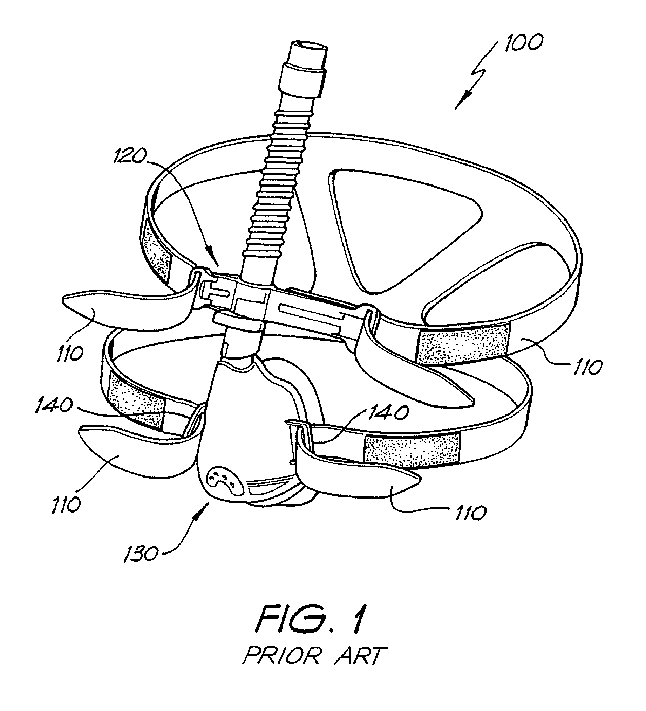

One known example is the Mirage® Mask (ResMed Limited), shown in Fig. 1. In this case, the headgear 100 is constructed from fabric and includes a rear portion, which engages the region near the occiput of the patient, and four straps 110 which are secured to a forehead support 120 (2 straps) and nasal mask frame 130 (2 straps). The straps include hook and loop material, such as Velcro(TM) on one side. The mask frame and forehead supports include loops 140 through which a strap can pass. In order to secure the mask in place on a patient's head, the four straps are passed through the four corresponding loops and held in place at an appropriate length by the hook and loop material. The patient can adjust the length of the material in order to secure a good fit of the mask. In some cases, it can require considerable adjustment to find the optimal length of straps which is required. A difficulty with this connector is that if the patient removes the mask by loosening the straps, they will lose what may have been a good fit of the mask the next time it is being used. In this case they will need to repeat the adjustment step.

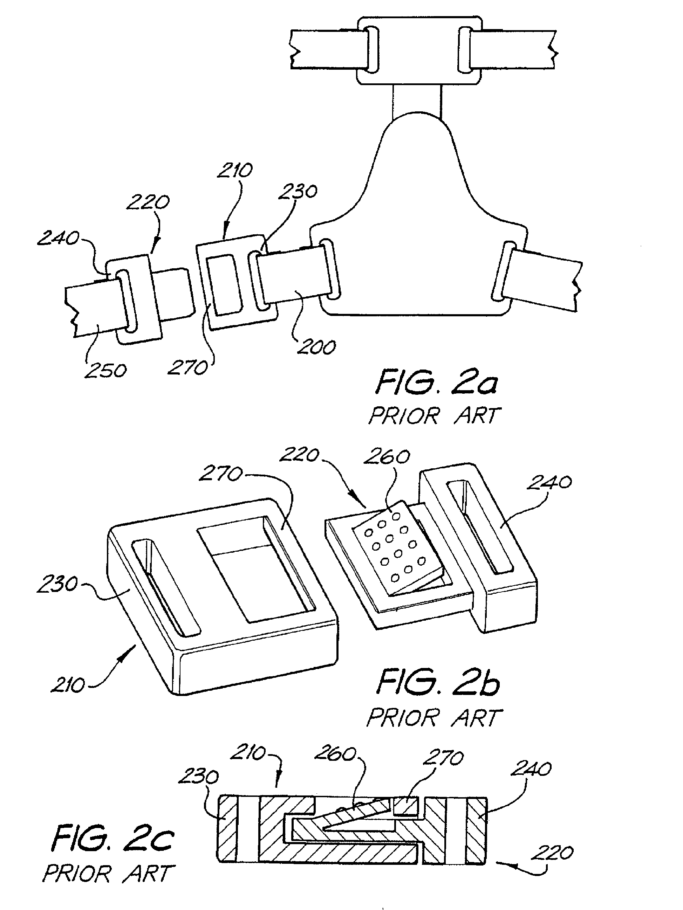

Another known manner of connecting the mask and headgear is shown in Figs. 2a, 2b and 2c. In this headgear, a short strap length 200 is secured to one side of the mask, with a two-part, press-release connector 210, 220 attaching this to the strap 250 of the headgear. One disadvantage of the approach of using this arrangement is that the connector may be difficult to release because the connector is free to move relative to the mask frame. A further disadvantage of this arrangement is that the connector may be in contact with the patient's face which may lead to discomfort in use, particularly if they sleep on their side.

More detailed views of this prior art connector are shown in Figs. 2b and 2c. Each part of the connector includes a bar 230, 240 behind which a respective one of the straps 200, 250 may be passed. The male portion 220 of the connector includes a resilient cantilever 260 which is captured behind a bar 270 on the female portion 210. The cantilever is depressed to engage and disengage the connector. A further disadvantage of this particular connector, best seen in Fig. 2b, is that it may be awkward to disengage the connector because of the close positioning of the bar 270 to the cantilever 260.

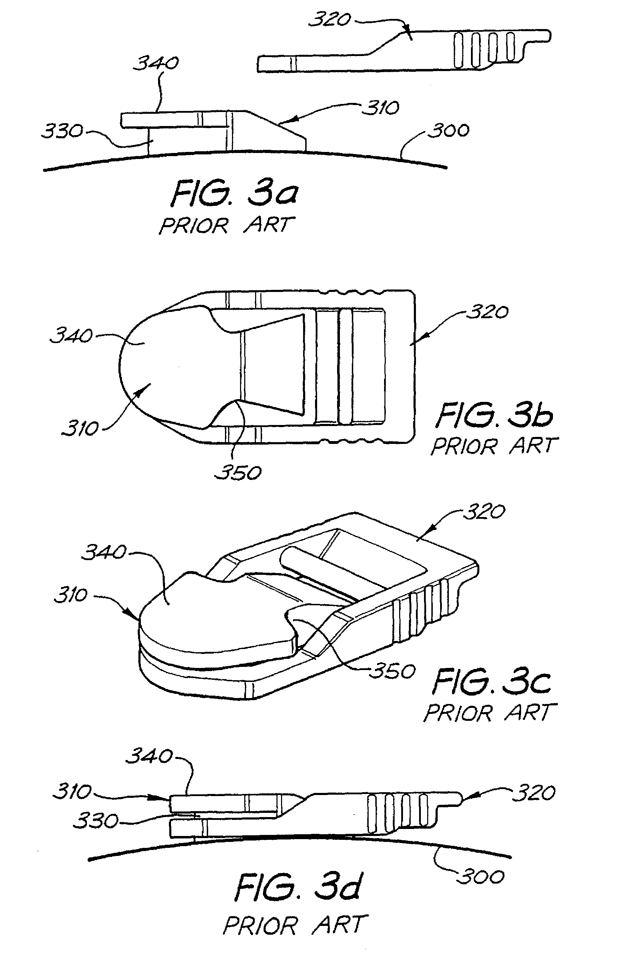

Another known mask and headgear connector is shown in Fig. 3a to Fig. 3d. This comprises a flexible part 310 positioned on the outer surface of a flexible mask frame 300 and a rigid part 320 formed generally as a D-ring with a loop to which the headgear strap is attached. The flexible part consists of a base 330 supporting an upper portion 340 (best illustrated in Fig. 3a) which overhangs the base portion 330 and has a narrow central region 350. In order to engage and disengage the connector, the flexible part 310 must be deformed whilst the D-ring of the rigid part is pushed over the upper portion 340 to engage below the overhanging ledge. This arrangement is awkward to engage and disengage and typically requires two hands.

Another known mask and headgear connector consists of hooks on the end of the headgear straps and corresponding holes in the mask frame. To engage the mask and headgear connector, the hook is passed through one of the corresponding holes on the headgear. This arrangement is also awkward to engage and disengage and typically requires two hands. Also, it is possible for the hooks to disengage during sleep as there is no locking means for the connection.

There is a need for a connector arrangement which is simple and quick to operate.

### Summary of the Invention

The invention provides, in one form, a respiratory mask and headgear combination comprising a respiratory mask having a rigid mask frame, adjustable headgear for securing said mask on a patient, said headgear including at least one attachment strap, said mask frame having rigidly secured thereto a rigid first connector, further comprising a second connector adapted for releasable mating with said first connector, said second connector having means for connection of said attachment strap of the headgear.

The invention further provides a respiratory mask and headgear combination adopted for single-handed disengagement, comprising a rigid mask frame, adjustable headgear for securing said mask on a patient, said headgear including at least one attachment strap, said mask frame having rigidly secured thereto a rigid first connector, further comprising a second connector connected to said strap of said headgear, said second connector being adapted for releasable mating with the first connector and having first and second gripping surfaces positioned for gripping of said second connector between a thumb and finger of a patient's hand and release means positioned for operation by another finger of the patient's hand.

### Brief Description of the Drawings

Further embodiments of the invention will now be described with reference to the accompanying drawings, in which:
Fig. 1 shows the prior art Mirage® mask and headgear assembly.
Fig. 2a shows a front view of a prior art mask and headgear assembly.
   Fig. 2b shows further detail of the prior art mask and headgear connector shown in Fig. 2a.
   Fig. 2c shows a cross-sectional view of the prior art mask and headgear connector shown in Fig. 2b.
Fig. 3 a shows a side view of another prior art mask and headgear connector where the two parts are not engaged.
   Fig. 3b shows a top view of the prior art connector shown in Fig. 3a, where the two parts are engaged.
   Fig. 3c shows a perspective view of the prior art connector shown in Fig. 3a, where the two parts are engaged.
   Fig. 3d shows a side view of the prior art connector shown in Fig. 3a, where the two parts are engaged.
Fig. 4 shows a front view of a mask frame incorporating a female portion of the connector according to one embodiment of the invention.
Fig. 5 shows a perspective view of the mask frame of Fig. 4.
Fig. 6a shows a front view of a male portion of the connector adapted for use with the mask frame of Figs. 4 and 5.
   Fig. 6b shows a side view of Fig. 6a.
   Fig. 6c shows a sectional view along A-A of Fig. 6a.
   Fig. 6d shows a perspective view of Fig. 6d.
Fig. 7a and 7b show an embodiment of the invention where the cross-bar on the male portions of the connectors have different angles.

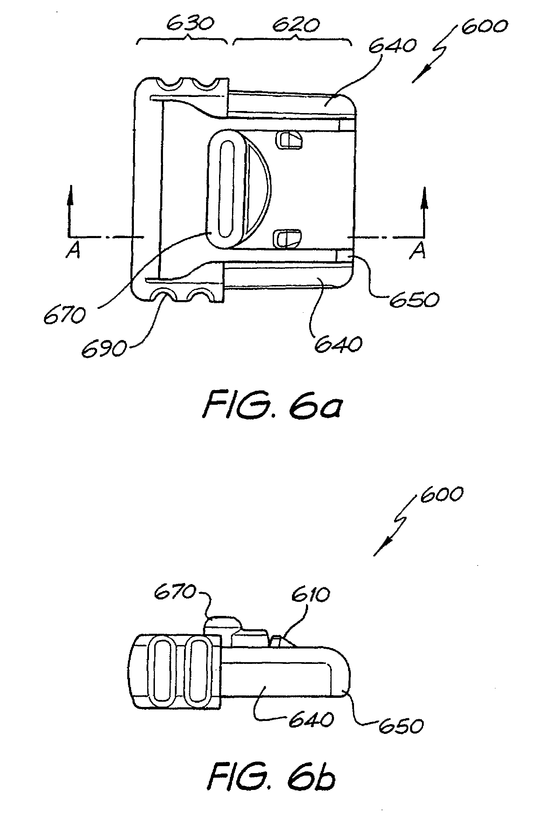

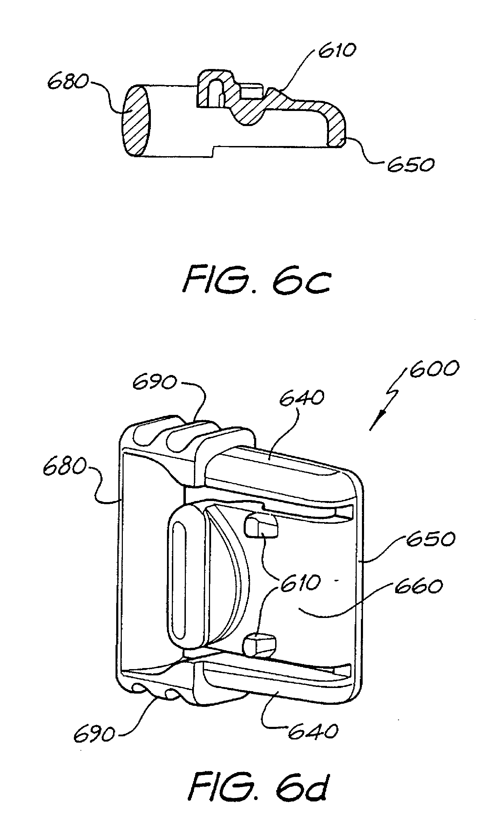

In Figs. 6a to 6d, dimensions shown are in millimeters.

### Detailed Description of the Invention

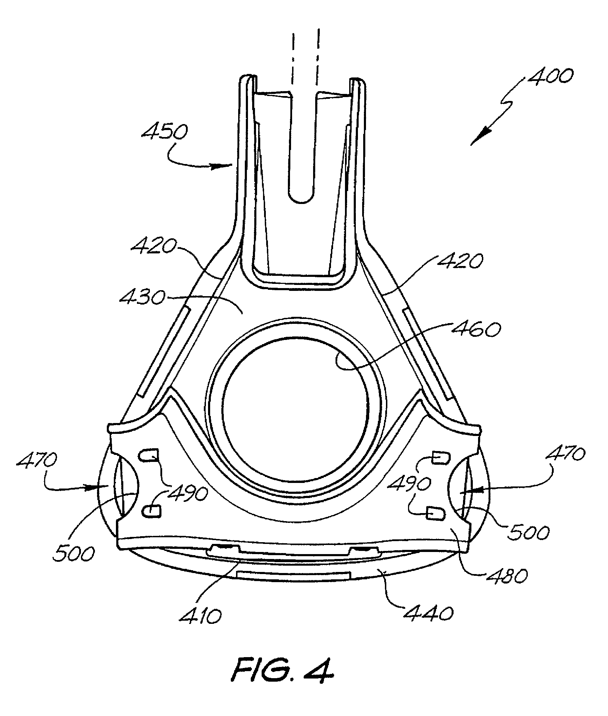

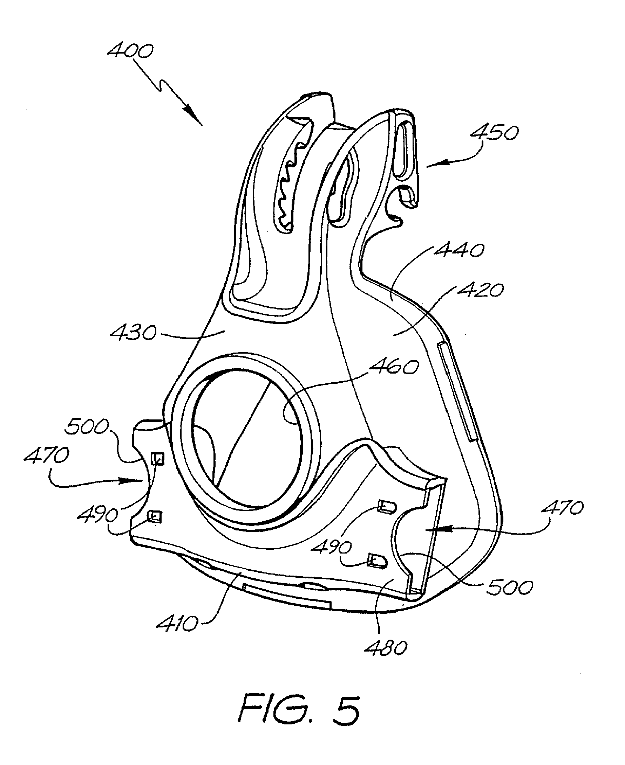

Figs. 4 and 5 illustrate a mask frame 400 for a nasal mask, formed as a moulded shell of polycarbonate or similar rigid material, which acts as a body onto which the other components of the mask are attached.

The frame 400 is generally triangular in front view, having a base 410, a pair of inclined side walls 420 extending towards an apex and a front wall 430. The frame defines a mask cavity covering the patient's nose, and is open at its rear. A rim 440 at the rear edge of the base 410 and side walls 420 approximates the contours of the patient's face and is adapted for attachment of a soft mask cushion (not shown) to space the frame away from the patient's face for sealing and comfort. The apex of the frame has an extension 450 for attachment of a forehead support (not shown).

In the illustrated mask frame, a gas inlet aperture 460 is formed in the front wall 430, for connection of a gas supply conduit or similar, which may include an elbow connector (not shown) pivotably connected to the frame. In other forms of mask, the gas inlet aperture may be formed at the apex of the frame.

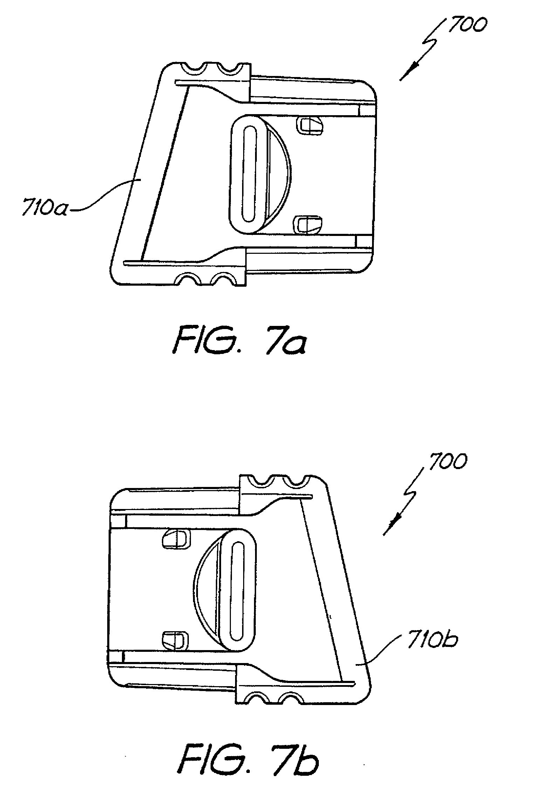

Integrally moulded into the lower corner regions of the rigid mask frame are a pair of female connector parts 470, for receiving the leading portions of the male connector parts 600, 700 illustrated in Figs. 6a to 6d, or Figs. 7a and 7b. These female connector parts open generally to the side of the mask, but are angled slightly downwards and rearwards (relative to the orientation of the mask frame) so that they hold the male connectors approximately parallel to that part of the patient's cheek region which the connectors overlie. The connectors are held clear of the patient's face by the rigid mask frame 400.

The female connectors 470 each define a recess which opens towards a side of the mask and which approximates a rounded-comered rectangular in end view, adapted to provide a close fit with the corresponding male connectors 600,700 when engaged. These end shapes are preferably slightly asymmetric (keyed) so as to prevent upside- down insertion of the male connector.

A front wall 480 of the female connector has a pair of sockets 490 at least on its inner surface, for receiving respective lugs 610 of the male connector 600. The end of the front wall may also have a curved cut-out portion 500, as will be described below.

A corresponding male connector part 600 is illustrated in Figs. 6a to 6d.

The male connector has a leading portion 620 which is received in the recess of the female connector 470, and a trailing portion 630 which remains outside the recess. The leading portion includes upper and lower side beams 640 connected by a cross-piece 650 at their leading ends, and a resiliently biased cantilever member 660 depending from the cross-piece 650 and extending back towards the trailing portion. The cantilever has on its front surface the lugs 610, and a ridge 670 at its trailing edge.

The locating lugs 610 have a rounded wedge profile so as to allow reduce wear and provide smooth engagement. The wedge profile does allow insertion of the male connector 600 into the female connector 470 on the mask without depression of the cantilever, although in preferred operation the patient will depress the cantilever by pushing on ridge 670 for both engagement and disengagement. Once the male connector has been inserted sufficiently to snap the locating lugs 610 into engagement with corresponding sockets 490 of the female connector, disengagement can occur only by depression of the cantilever.

The trailing portion 630 of the male connector has a cross-bar 680 forming a loop through which the headgear strap can be passed and adjusted for proper fit. Upper and lower sides 690 of the trailing portion have grooves or other formations to make it more easily gripped by the patient's fingers, while the distance between the leading side of the cross-bar 680 and the trailing edge and ridge 670 of the cantilever is preferably at least 5mm to allow easy connection of the strap and provide sufficient space for a finger to push on the cantilever ridge, even when the strap is attached. The arcuate cut-out 500 in the female connector front wall allows clearance for an end of the finger to overhang the ridge 670 when the ridge is being contacted by the pad of the finger.

Figs 7a and 7b illustrate a male connector 700 which is a variation on that of Figs 6a to 6d, in that the cross bars 710a, 710b for attachment of the headgear strap are set at an angle.

The shape, dimensions and position of the connectors may be optimised for most convenient use. For example, the male connector may be approximately 20 mm wide, 25 mm long and 10 mm thick. The cantilever 660 is approximately 15 mm long. The locating lugs 610 are approximately 3 mm long, 2 mm wide and 1 mm high, and the ridge is approximately 14 mm wide and has a length of approximately 5 mm. Other dimensions of the male portion are indicated in Fig. 6a to 6c.

The female connectors 470 are oriented on the mask frame so that in use the straps of the headgear are aligned to be approximately parallel with the sides of the face with which they make contact. When viewed from the front of the mask (the view in Fig. 4), the tops of the left and right hand side male connector's cross-bars 680 are further apart than the bottoms of the cross-bars. When viewed from the top the mask, the connectors are aligned to be positioned on an arc of an ellipse. An advantage from this arrangement is that when straps are connected and tensioned, the line of force will be more evenly transferred from the mask to the frame.

Both portions of the connector are preferably constructed from polycarbonate, such as Makrolon 2458 polycarbonate from Bayer, or similar rigid plastics material. The female portions of the connectors are clear and desirably are integrally moulded with the mask frame which is formed of the same polycarbonate material, thus ensuring a permanent, rigid attachment of the female connector to the mask frame and minimising the number of separate parts. The male portions may be frosted or textured. An advantage from using a frosted or slightly textured surface is that the male portion may be easier to distinguish from the female portion by touch. This is an advantage in the dark, the typical time when the mask is being used by a patient.

The configuration thus allows single-hand operation of the connector using, for example, thumb and index finger to grip the grooved sides 690 of the male connector and the middle finger to depress the cantilever. The connectors may be located on both the left and right hand side of the mask. In this way persons may use which ever side is most convenient to them. Alternatively the frame may be formed with only one connector, to reduce manufacturing costs.

In one unillustrated embodiment, the press release mechanism may be adapted to be operated by pressing the top and/or bottom sides of the male connector.

By rigidly forming one part of the connector onto the mask frame, the patient does not need to use both hands to disconnect the mask for removal. Thus, the arrangement facilitates quick and easy disconnection even though the connector will be out of patient's line of vision and the patient may be less than fully awake or in an anxious state.

A further advantage of using female portions 470 integrated with the mask frame is that they result in a reduced dead volume of the mask frame.

While particular embodiments of this invention have been described, it will be evident to those skilled in the art that the present invention may be embodied in other specific forms without departing from the essential characteristics thereof. The present embodiments and examples are therefore to be considered in all respects as illustrative and not restrictive, the scope of the invention being indicated by the appended claims rather than the foregoing description, and all changes which come within the meaning and range of equivalency of the claims are therefore intended to be embraced therein.

## Claims

1. A respiratory mask and headgear combination comprising a respiratory mask having a rigid mask frame (400), adjustable headgear for securing said mask on a patient, said headgear including at least one attachment strap, whereby said mask has a first connector portion (470), and a second connector portion (600) adapted for releasable mating with said first connector portion (470), said second connector portion (600) having a cross bar forming a loop through which the attachment strap can be passed and adjusted for proper fit, wherein said second connector portion (600) is at least partially insertable into the first connector portion (470) to form a snap-fit locked connection between the first and second connector portion, and wherein the mask includes a press-release mechanism having at least one depressible portion to allow single-handed disengagement by the patient in use.

2. A respiratory mask and headgear combination according to claim 1, wherein first connector portion (470) is a female connector portion.

3. A respiratory mask and headgear combination according to claim 1 or 2, wherein said second connector portion is a male connector portion (600).

4. A combination according to any preceding claim, in which said first connector portion (470) is integrated with said mask frame (400).

5. A combination according to any preceding claim, wherein two first connector portions (470) are provided, each at a lower corner portion of the mask frame (400).

6. A combination according to any preceding claim, wherein the first connector portions (470) are angled downwards and/or rearwards relative to the orientation of the mask frame (400).

7. A combination according to any preceding claim, wherein the first connector portions (470) are orientated on the mask frame (400) such that in use the straps of headgear connecting with the connector portions are aligned to be approximately parallel with the sides of a users face.

8. A respiratory mask and headgear combination according to any preceding claim, wherein said press-release mechanism is associated with a resiliently biased cantilever member (660) having means (610) for releasable locking engagement between the connector portions (600, 470).

9. A respiratory mask and headgear combination according to claim 8, wherein said cantilever member (660) has a leading end, a trailing end, locking means (610) located intermediate said leading end and trailing end portion (470), and release means (670) located adjacent said trailing end.

10. A respiratory mask and headgear combination according to claim 9, wherein said release means (670) comprises a raised portion adjacent a trailing end of said cantilever member.

11. A respiratory mask and headgear combination according to claim 10, wherein a space is provided immediately behind said trailing end of the cantilever member.

12. A respiratory mask and headgear combination according to any one of claims 9 to 11, wherein said locking means comprises at least one lug (610) on a forward surface of said cantilever member, said lug engaging a corresponding socket (490).

13. A respiratory mask and headgear combination according to any one of claims 1 to 12, wherein said first and second connector portions are spaced forwardly of the patient's face by said rigid mask frame.

14. A respiratory mask and headgear combination according to any one of claims 1 to 3 and 9, wherein said press-release mechanism includes a pair of lugs (610) mounted on a flexible cantilever member (660) and a pair of sockets (490) structured to selectively release the respective lugs.

15. A respiratory mask and headgear combination according to any preceding claim, wherein said first connector portion is rigidly secured to said mask frame.
